# EUROPÄISCHE PATENTSCHRIFT

(11) **EP 0 809 109 B1**
(45) Veröffentlichungstag und Bekanntmachung des Hinweises auf die Patenterteilung: **17.05.2006**
(21) Anmeldenummer: 97103578.7
(22) Anmeldetag: 05.03.1997
(51) Int. Cl.: G01N 33/00, G01N 27/62, G01M 15/00

(54) **Vorrichtung zur Messung von Brennkraftmaschinen-Abgas-Komponenten**
Apparatus for measuring combustion machine exhaust gas components
Appareil pour la mesure de composants de gaz d'échappement d'une machine à combustion

(30) Priorität: 25.05.1996 DE 19621293
(43) Veröffentlichungstag der Anmeldung: 26.11.1997
(73) Patentinhaber: Pierburg Instruments GmbH, 41460 Neuss (DE)
(72) Erfinder: Garthe, Christopher, 40237 Düsseldorf (DE)
(74) Vertreter: Ter Smitten, Hans

(56) Entgegenhaltungen:
- EP-A- 0 681 182
- WO-A-96/06349
- DE-A- 4 131 412
- US-A- 4 981 652
- US-A- 5 221 517

## Beschreibung

Die Erfindung betrifft eine Vorrichtung zur Messung von Brennkraftmaschinen-Abgaskomponenten nach dem Oberbegriff des Patentanspruchs 1.

Eine derartige Vorrichtung ist in der Druckschrift Emissions- und Immisionstechnik im Verkehrswesen,G, Hauschulz, Köln, Verlag TÜV-Rheinland, 1993, Seiten 214 und 215 beschrieben. Das Meßprinzip eines dort offenbarten Flammenionisationsdetektors beruht darauf, daß in einer Wasserstoffflamme aus den Kohlenwasserstoffmolekülen Ionen gebildet werden. Die Flamme brennt zwischen zwei Elektroden, an denen eine Gleichspannung anliegt. Brennluft und Brenngas werden dem Brenner zugeführt. Die Abgasprobe wird dem Brenngas vor der Brennerdüse zugemischt. Die in der Flamme entstehenden Ionen bewerkstelligen einen Ladungstransport, der als Ionenstrom meßbar ist. Das in der Druckschrift offenbarte Schaltschema des Flammenionisationsdektors zeigt diverse Anschlußleitungen für Probe-, Test- und Betriebsgase, in denen jeweils ein Präzisionsdruckregler und eine Ablaufdüse angeordnet sind, um einen festen Durchfluß der einzelnen Komponenten zu erzielen. Eine ähnliche Vorrichtung ist in der US-Patentschrift US 5 221 517 offenbart (Methan-Analysator). Damit die Vorrichtung genau arbeitet, muß der Druckabfall an den Ablaufdsen genau eingehalten werden, was nur mit teueren Druckreglern zu erzielen ist. Da jeder Druckregler einer Exemplarstreuung unterliegt, kann die Vielzahl der Druckregler insgesamt zu Ungenauigkeit der Meßergebnisse führen, sie stellt jedoch auch einen Verteuerungsfaktor dar.

Aus der erstgenannten Druckschrift, Seite 312, geht hervor, daß sich die im Abgas enthalte-nen Kohlenwasserstoffanteile aus einer großen Zahl von Einzelkomponenten zusammensetzen, zu der auch Methan (CH4) gehört, dessen Beitrag zur Smogbildung relativ gering ist und deshalb bei der Bewertung entsprechend gering beurteilt werden sollte (Methan-Bonus).

Nach dieser Druckschrift können bestimmte Oxidationskatalysatoren so gesteuert werden, daß CH4 kaum angegriffen wird, wohingegen die anderen HC-Verbindungen zu CO2 konvertiert werden. Somit kann CH4 und Gesamt-HC getrennt in einem Flammenionisationsdetektor festgestellt werden und durch Differenzbildung die methanfreie HC-Emission.

Die chemische Oxidation der Kohlenwasserstoffe im Abgas erfordert die gleichzeitige Anwesenheit des Reaktionspartners Sauerstoff im Meßgas. Ist das Meßgas /Abgas aus Kraftfahrzeugmotoren, dann ist der Sauerstoffgehalt nicht ausreichend. Der für die Umset zung notwendige Sauerstoff wird dem Abgas über sogenannte Brennluftanteile zugeführt. Die im Abgas auftretenden Kohlenwasserstoffkonzentrationen sind jedoch für übliche Konverter zu hoch. Es ist somit eine Verdünnung des Abgases erforderlich, wobei das Verdünnungsverhältnis Brennluft zu Abgas genau bestimmt sein muß, um die Gesamtemission bestimmen zu können. Hierfür wäre zusätzlich ein Kostenmehraufwand für Druckregler ect. notwendig.

Hiervon ausgehend liegt der Erfindung daher die Aufgabe zugrunde, eine angegebene Vorrichtung derart zu gestalten, daß gegenüber bekannten Systemen eine Vereinfachung und Reduzierung von Bauteilen, insbesondere von Druckreglern, erreichbar werden, darüber hinaus soll eine Verdünnung des Abgases erzielt werden, die von möglichst wenigen Einflußparametern abhängt.

Diese Aufgabe ist durch die im Patentanspruch 1 angegebenen Merkmale gelöst worden. Vorteilhafte Weiterbildungen nennen die Unteransprüche.

Zwei Ausführungsbeispiele der Erfindung sind in der Zeichnung dargestellt und werden nachfolgend beschrieben.

Es zeigt:
Fig. 1
   eine prinzipielle Ausführung als Teil einer Vorrichtung und
Fig. 2
   eine spezielle Ausführung einer Vorrichtung zur Messung von HC- und HC4-Anteilen im Abgas.

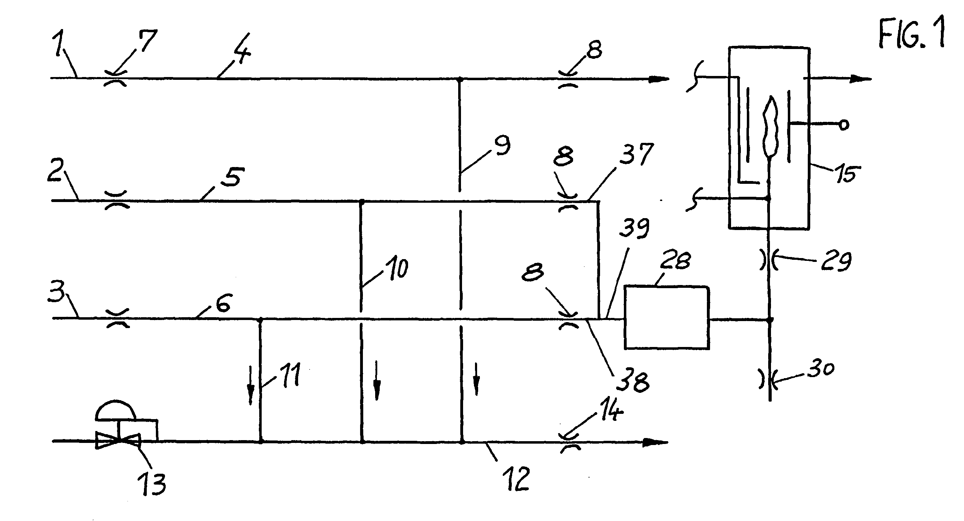

Fig. 1 zeigt diverse Anschlußleitungen 1, 2, 3 für Probe-, Test- und Betriebsgase als Teil einer Vorrichtung zur Messung von Brennkraftmaschinen-Abgaskomponenten in Verbindung mit einem nicht dargestellten Gasanalysator. Diese Anschlußleitungen weisen jeweils einen Leitungsabschnitt 4, 5, 6 zwischen einer Zulauf- und einer Ablaufdüse 7 und 8 auf, die über Verbindungsleitungen 9, 10, 11 mit einem Steuerluftleitungsabschnitt 12 verbunden sind, der zwischen einem Druckregler 13 und einer mit der Atmosphäre verbundenen Bypaßdüse 14 liegt. Es ist vorgesehen, daß der Durchsatz der Zulaufdüsen 7 größer als der der Ablaufdüsen 8 ist, so daß sich ein Durchsatz über die Verbindungsleitungen 9, 10, 11 in die Steuerluftleitung 12 einstellt. Durch diese Maßnahme wird erreicht, daß zusätzlich zu dem Druck in der Steuerluftleitung 12 auch der in den anderen Leitungsabschnitten 4, 5, 6 herrschende Druck auf das Druckniveau des Druckreglers 13 der Steuerluftleitung 12 geregelt wird. Damit ist der Aufwand für bisher übliche Druckregler nicht mehr notwendig.

Der aus den einzelnen Leitungsabschnitten 4, 5, 6 über die Verbindungsleitungen 9, 10, 11 in die Steuerluftleitung 12 gelangende Gasanteil ist äußerst gering und tritt über die Bypaßdüse 14 in die Atmosphäre aus. Da an allen Ablaufdüsen 8 der Anschlußleitungen 1, 2, 3 der gleiche Druck ansteht, läßt sich der Düsendurchsatz genau einstellen. Aus allen Gasarten läßt sich ein wohldefiniertes Durchsatzverhältnis der betreffenden Gase bilden, wenn diese zusammengeführt werden, wobei der an den Ablaufdüsen 8 anstehende Druck für das Durchsatz-Mischungsverhältnis unkritisch ist, dieser bestimmt lediglich den Durchfluß, d.h. Druckschwankungen in gewissen Grenzen sind ohne Auswirkungen.

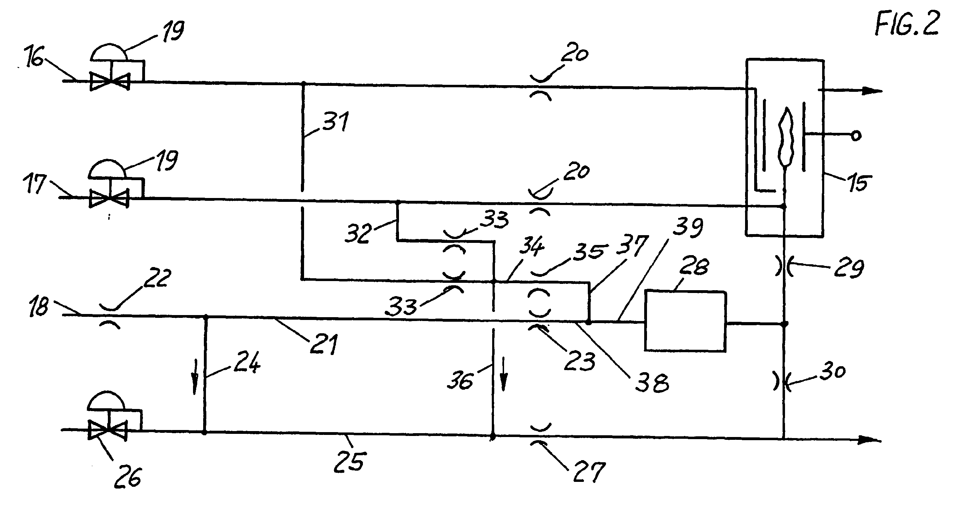

Fig. 2 zeigt eine Vorrichtung mit einem Flammenionisationsdetektor 15, bestehend aus Anschlußleitungen 16, 17, 18 für Brennluft, Brenngas und Abgas. Die Leitungen 16, 17 weisen einen Druckregler 19 und eine Ablaufdüse 20 auf und münden in den Flammenionisationsdetektor 15 ein.

Die Abgasleitung 18 weist einen Leitungsabschnitt 21 zwischen einer Zulaufdüse 22 und einer Ablaufdüse 23 auf, der über eine Verbindungsleitung 24 mit einem Steuerluftleitungsabschnitt 25 verbunden ist, der zwischen einem Druckregler 26 und einer mit der Atmosphäre verbundenen Bypaßdüse 27 liegt, wobei auch hier der Durchsatz der Zulaufdüse 22 größer als der der Ablaufdüse 23 ist, so daß sich ein Durchsatz über die Verbindungsleitung 24 in die Steuerluftleitung 25 einstellt.

Die Abgasleitung 18 ist stromab der Ablaufdüse 23 mit einem Konverter 28 verbunden, von dem die Abgasleitung 18 über eine weitere Düse 29 in den Flammenionisationsdetektor 15 einmündet und über eine Bypaßdüse 30 in den stromab der Bypaßdüse 27 liegenden Teil der Steuerluftleitung 25 oder alternativ direkt in die Atmosphäre. Dem Abgas wird für die Konvertierung der Kohlenwasserstoffe benötigte Brennluft und ggf. auch Brenngas zugeführt, d.h. das Abgas wird in einem festen Verhältnis mit Brennluft verdünnt, der zuvor ein geringer Anteil Brenngas zugemischt wurde.

Hierfür weist die Vorrichtung von den Anschlußleitungen 16, 17 zwischen den Druckreglern 19 und den Ablaufdüsen 20 abzweigende Abzweigleitungen 31, 32 mit Zulaufdüsen 33 auf, die stromab der Zulaufdüsen 33 einen gemeinsamen Leitungsabschnitt 34 mit einer Ablaufdüse 35 bilden, der über eine Verbindungsleitung 36 mit dem Steuerluftleitungsabschnitt 25 zwischen dem Druckregler 26 und der Bypaßdüse 27 verbunden ist.

Der gemeinsame Leitungsabschnitt 34 mündet stromab der Ablaufdüse 35 in einen zwischen Ablaufdüse 23 und Konverter 28 bestehenden Abgasleitungsabschnitt 39 ein. An diesem gemeinsamen Austrittspunkt herrscht natürlich der gleiche Druck. Um zu gewährleisten, daß das Verhältnis von Abgas zu Luft gleich bleibt, muß auch der Druck vor den Ablaufdüsen 23, 35 gleich bleiben. Um diese Voraussetzungen zu erfüllen, verwendet man die erfinderischen Maßnahmen, daß die Ablaufdüse 35 des gemeinsamen Leitungsabschnitts 34 und die Ablaufdüse 23 der Abgasleitung 21 mit dem von dem Druckregler 26 der Steuerluftleitung 25 eingestellten Druck beaufschlagt sind. Durch die gleichen Drücke am Eingang wie am Ausgang der Ablaufdüsen 23 und 35 wird also immer in gleichen Verhältnissen Abgas und Luft miteinander gemischt. Dabei ist es unerheblich, ob der Druck am Ausgang der Ablaufdüsen 23, 35 sich verändert. Selbst der geregelte Druck an den Eingängen der Ablaufdüsen kann sich verändern, wenn z. B. der Druckregler 26 nicht genau arbeitet, ohne daß das Mischungsverhältnis verändert wird. Somit läßt sich auch hier ein wohldefiniertes Durchsatz-Mischungsverhältnis zwischen Abgas und Brennluftgas bilden, das für die Konzentrationsbestimmung konstant und reproduzierbar sein muß.

In einfachster Ausführung kann vorgesehen sein, daß wenigstens zwei der stromab der Ablaufdüsen 8 bzw. 23, 35 liegenden Leitungsabschnitte 37, 38 in einen gemeinsamen Leitungsabschnitt 39 einmünden, der einen Mischabschnitt für die einmündenden Gase bildet.

## Patentansprüche

1. Vorrichtung zur Messung von Brennkraftmaschinen-Abgaskomponenten in Verbindung mit einem Flammenionisationsdetektor, enthaltend diverse Anschlußleitungen für Probe-, Test-und Betriebsgase, **dadurch gekennzeichnet, daß** wenigstens zwei Anschlußleitungen jeweilige Leitungsabschnitte (4, 5, 6) zwischen jeweils einer Zulauf- und jeweils einer Ablaufdüse (7 und 8) aufweisen, die über Verbindungsleitungen (9, 10, 11) mit einem Steuerleitungsabschnitt (12) verbunden sind, der zwischen einem Druckregler (13) und einer mit der Atmosphäre verbundenen Bypaßdüse (14) liegt, wobei der Durchsatz der jeweiligen Zulaufdüsen (7) größer als der der jeweiligen Ablaufdüsen (8) ist, so daß sich ein Durchsatz über die Verbindungsleitungen (9, 10, 11) in den stenerleitungsabsdnitt (12) einstellt.

2. Vorrichtung nach Anspruch 1, **dadurch gekennzeichnet, daß** mehrere Anschlußleitungen (16, 17) über Abzweigleitungen (31, 32) mit Zulaufdüsen (33) einen gemeinsamendieser Leitungsabschnitte (34) mit der Ablaufdüse bilden.

3. Vorrichtung nach Anspruch 1 oder. 2, **dadurch gekennzeichnet, daß** wenigstens zwei der stromab der Ablaufdüsen (8 bzw. 23, 35) liegenden Leitungsabschnitte (37, 38) in einen gemeinsamen Leitungsabschnitt (39) einmünden, der einen Mischabschnitt für die einmündenden Gase bildet.

## Claims

1. Apparatus for measuring internal combustion engine exhaust gas components in conjunction with a flame ionization detector, containing various feed lines for sample, test and fuel gases, **characterised in that** at least two feed lines comprise respective line portions (4, 5, 6) between a respective supply nozzle and a respective discharge nozzle (7 and 8), which line portions are connected via connection lines (9, 10, 11) to a control line portion (12) which lies between a pressure regulator (13) and a bypass nozzle (14) communicating with the atmosphere, wherein the throughput of the respective supply nozzles (7) is greater than that of the respective discharge nozzles (8), so that a throughput is set via the connection lines (9, 10, 11) into the control line portion (12).

2. Apparatus according to Claim 1, **characterised in that** a plurality of feed lines (16, 17) form a common one of these line portions (34) with the discharge nozzle via branch lines (31, 32) with supply nozzles (33).

3. Apparatus according to Claim 1 or 2, **characterised in that** at least two of the line portions (37, 38) lying downstream of the discharge nozzles (8, respectively 23, 35) lead into a common line portion (39) which forms a mixing portion for the inflowing gases.

## Revendications

1. Dispositif de mesure de constituants de gaz d'échappement de moteurs à combustion interne en relation avec un détecteur à ionisation de flamme, comprenant diverses conduites de raccordement pour des gaz d'échantillonnage, gaz d'essai et gaz à usage interne, **caractérisé en ce qu'**au moins deux conduites de raccordement présentent chacune une section de conduite (4, 5, 6) entre une buse d'admission et une buse de sortie (7 et 8) respectives, lesquelles sections de conduite sont raccordées par l'intermédiaire de conduites de jonction (9, 10, 11) à une section de conduite de réglage (12) située entre un détendeur (13) et une buse de bipasse (14) reliée à l'atmosphère, le débit des buses d'admission respectives (7) étant supérieur à celui des buses de sortie respectives (8) de sorte qu'un débit se règle dans la section de conduite de réglage (12) par l'intermédiaire des conduites de jonction (9, 10, 11).

2. Dispositif suivant la revendication 1, **caractérisé en ce que** plusieurs conduites de raccordement (16, 17) forment par l'intermédiaire de conduites de dérivation (31, 32) avec des buses d'admission (33) une section de conduite commune (34) avec la buse de sortie.

3. Dispositif suivant l'une des revendications 1 et 2, **caractérisé en ce qu'**au moins deux des sections de conduite (37, 38), situées en aval des buses de sortie (8 ou 23, 35), débouchent dans une section de conduite commune (39) qui forme une section de mélange pour les gaz admis.
